# EUROPEAN PATENT SPECIFICATION

(11) **EP 2 701 780 B1**
(45) Date of publication and mention of the grant of the patent: **30.03.2016**
(21) Application number: 12717691.5
(22) Date of filing: 26.04.2012
(51) Int. Cl.: A61M 5/34, A61M 5/50

(54) **DISPENSE INTERFACE WITH LOCKOUT ELEMENT**
AUSGABESCHNITTSTELLE MIT AUSSPERRELEMENT
INTERFACE D'ADMINISTRATION AYANT UN ÉLÉMENT DE VERROUILLAGE

(30) Priority: 28.04.2011 US 201161480063 P; 08.07.2011 EP 11173271
(43) Date of publication of application: 05.03.2014
(73) Proprietor: Sanofi-Aventis Deutschland GmbH, 65929 Frankfurt am Main (DE)
(72) Inventor: EGGERT, Ilona, 65926 Frankfurt am Main (DE); DAVIES, James, Alexander, Leamington Spa Warwickshire CV32 7XB (GB); BILTON, Simon, Lewis, Leamington Spa Warwickshire CV32 5QT (GB); MOORE, David, Leicester Leicestershire LE9 7SA (GB); WIMPENNY, Steven, Leamington Spa Warwickshire CV32 6ES (GB); LANGLEY, Christopher, Nigel, Leamington Spa Warwickshire CV32 7HH (GB)
(74) Representative: McDougall, Robert Campbell
(86) International application number: PCT/EP2012/057685
(87) International publication number: WO 2012/146673

(56) References cited:
- US-A1- 2008 154 192

## Description

The present patent application relates to medical devices for delivering at least two drug agents from separate reservoirs. Such drug agents may comprise a first and a second medicament. The medical device includes a dose setting mechanism for delivering the drug agents automatically or manually by the user.

The medical device can be an injector, for example a hand-held injector, especially a pen-type injector, that is an injector of the kind that provides for administration by injection of medicinal products from one or more multidose cartridges. In particular, the present invention relates to such injectors where a user may set the dose.

The drug agents may be contained in two or more multiple dose reservoirs, containers or packages, each containing independent (single drug compound) or pre-mixed (coformulated multiple drug compounds) drug agents.

Certain disease states require treatment using one or more different medicaments. Some drug compounds need to be delivered in a specific relationship with each other in order to deliver the optimum therapeutic dose. The present patent application is of particular benefit where combination therapy is desirable, but not possible in a single formulation for reasons such as, but not limited to, stability, compromised therapeutic performance and toxicology.

US2008/154192 A1 discloses a needle tip assembly comprising: a needle tip; and at least one of: an arrangement for preventing re-installation or re-use of the needle tip; a safety mechanism coupled to the needle tip via a living hinge; and a safety cover non-removably coupled to the needle tip. The preamble of claim 1 is based on the disclosure of said document.

For example, in some cases it may be beneficial to treat a diabetic with a long acting insulin (also may be referred to as the first or primary medicament) along with a glucagon-like peptide-1 such as GLP-1 or GLP-1 analog (also may be referred to as the second drug or secondary medicament).

Accordingly, there exists a need to provide devices for the delivery of two or more medicaments in a single injection or delivery step that is simple for the user to perform without complicated physical manipulations of the drug delivery device. The proposed drug delivery device provides separate storage containers or cartridge retainers for two or more active drug agents. These active drug agents are then combined and/or delivered to the patient during a single delivery procedure. These active agents may be administered together in a combined dose or alternatively, these active agents may be combined in a sequential manner, one after the other.

The drug delivery device also allows for the opportunity of varying the quantity of the medicaments. For example, one fluid quantity can be varied by changing the properties of the injection device (e.g., setting a user variable dose or changing the device's "fixed" dose). The second medicament quantity can be changed by manufacturing a variety of secondary drug containing packages with each variant containing a different volume and/or concentration of the second active agent.

The drug delivery device may have a single dispense interface. This interface may be configured for fluid communication with a primary reservoir and with a secondary reservoir of medicament containing at least one drug agent. The drug dispense interface can be a type of outlet that allows the two or more medicaments to exit the system and be delivered to the patient.

The combination of compounds from separate reservoirs can be delivered to the body via a double-ended needle assembly. This provides a combination drug injection system that, from a user's perspective, achieves drug delivery in a manner that closely matches the currently available injection devices that use standard needle assemblies. One possible delivery procedure may involve the following steps:
1. Attach a dispense interface to a distal end of the electro-mechanical injection device. The dispense interface comprises a first and a second proximal needle. The first and second needles pierce a first reservoir containing a primary compound and a second reservoir containing a secondary compound, respectively.
2. Attach a dose dispenser, such as a double-ended needle assembly, to a distal end of the dispense interface. In this manner, a proximal end of the needle assembly is in fluidic communication with both the primary compound and secondary compound.
3. Dial up/set a desired dose of the primary compound from the injection device, for example, via a graphical user interface (GUI).
4. After the user sets the dose of the primary compound, the micro-processor controlled control unit may determine or compute a dose of the secondary compound and preferably may determine or compute this second dose based on a previously stored therapeutic dose profile. It is this computed combination of medicaments that will then be injected by the user. The therapeutic dose profile may be user selectable. Alternatively, the user can dial or set a desired dose of the secondary compound.
5. Optionally, after the second dose has been set, the device may be placed in an armed condition. The optional armed condition may be achieved by pressing and/or holding an "OK" or an "Arm" button on a control panel. The armed condition may be provided for a predefined period of time during which the device can be used to dispense the combined dose.
6. Then, the user will insert or apply the distal end of the dose dispenser (e.g. a double ended, needle assembly) into the desired injection site. The dose of the combination of the primary compound and the secondary compound (and potentially a third medicament) is administered by activating an injection user interface (e.g. an injection button).

Both medicaments may be delivered via one injection needle or dose dispenser and in one injection step. This offers a convenient benefit to the user in terms of reduced user steps compared to administering two separate injections.

Delivering one or more medicaments through a dose dispenser with a dispense interface can result in the contamination of the dispense interface with traces of each medicament. This contamination may prohibit reusing the dispense interface, for example after a certain time or after a predetermined number of usages, because the purity of the delivered medicaments cannot be guaranteed. Even a user who is conscious of this problem may inadvertently try to reuse a dispense interface because he may not remember and may find it difficult or impossible to determine by inspection whether a given dispense interface has in fact been used or not.

It is therefore desirable to provide the dispense interface with a mechanism that prevents reuse of the dispense interface with a drug delivery device. This mechanism should be such that it is effective in its prevention of reuse as well as safe from manual manipulation by a user.

The invention faces the technical problem of providing a dispense interface for use with a drug delivery device which is prevented of being reused after it has already been used with a drug delivery device.

This object has been solved by a dispense interface for use with a drug delivery device with an inner body and with a lockout element, wherein the lockout element is coupled to the inner body, wherein the lockout element is movable from a receptive condition to a locked condition, wherein in the receptive condition the dispense interface is attachable to the drug delivery device, wherein in the locked condition the dispense interface is not-attachable to the drug delivery device and wherein the lockout element is configured to move from the receptive condition to the locked condition when said dispense interface is attached to and detached from said drug delivery device.

The lockout element is arranged in its receptive condition such that it allows attachment of the dispense interface to the drug delivery device. However, the process of attaching the dispense interface to the drug delivery device mechanically moves the lockout element such that, once the dispense interface is detached and thereby is removed from the drug delivery device, the lockout element mechanically blocks a reattachment of the dispense interface to any drug delivery device. Therefore a reuse of the dispense interface is prevented and the risk of contamination from residual drug components within the dispense interface eliminated.

According to an advantageous embodiment of the dispense interface, the lockout element is movable from the receptive condition to an activated condition, wherein in the activated condition the lockout element is configured to move automatically to the locked condition when said dispense interface is detached from said drug delivery device, and wherein the lockout element is configured to move from the receptive condition to the activated condition when said dispense interface is attached to said drug delivery device. This embodiment ensures in a particular safe and reliable manner the lockout element to move from the receptive condition to the locked condition, when said dispense interface is attached to and detached from said drug delivery device.

Preferably, the lockout element has at least a spring element, which is strained in the receptive and/or the activated condition and at least partly relaxed in the locked condition. Accordingly, in the receptive and/or the activated condition the tensioned spring element stores energy, wherein in the locked condition the spring element stores less or no energy. In this configuration, the energy in the spring element is in a simple manner transformable into movement of the lockout element, especially an automatic movement to the locked condition. In particular, the spring element may effect an automatic movement of the lockout element from the activated to the locked condition.

The spring element may be an integral part of the lockout element or a separate element, which is connected to the lockout element. Further, the spring element may comprise one or more spring arms, whereas the spring element preferably comprises two spring arms.

It is further preferred, that the lockout element has at least a bearing section for bearing a distal portion of the drug delivery device, wherein in the receptive condition the bearing section is in an open position, in which it allows the distal portion of the drug delivery device to approach the inner body, and wherein in the locked condition the bearing section is in a blocking position, in which it prevents the distal portion of the drug delivery device to approach the inner body.

Providing a lockout element with a bearing section for bearing a distal portion of the drug delivery device, allows the reattachment of the dispense interface to be prevented mechanically with a particularly simple constructive design of the lockout element. At the same time the mechanical blocking is reliable and therefore safely prevents the dispense interface from reattachment. The blocking function may be further improved by providing more than one bearing section, in particular two bearing sections for bearing two distal portions of the drug delivery device.

According to a further embodiment of the dispense interface, the lockout element has at least a support section, wherein a support surface is provided on the inner body or an outer body, which is attached to the inner body, and wherein the lockout element is configured such that in the locked condition the support section is in engagement with the support surface so as to prevent the bearing section from being moved into the open position.

Thereby, the bearing section may reliably be maintained in the blocking position once the lockout element has moved into the locked condition, thus safely preventing the dispense interface from being reattached to the drug delivery device after it has been used. In particular, this embodiment ensures the bearing section not to be moved from the blocking position back to the open position due to geometrical restrictions. The support section may be a surface portion or an edge portion of the lockout element. A further increased blocking safety may be achieved by providing a lockout element with two or more support sections and an outer body or respectively an inner body with two or more corresponding support surfaces.

It is moreover preferred, that the bearing section is resiliently supported on the inner body by the spring element. Thereby, it is in a particularly simple manner possible to influence the strain condition of the spring element by attaching the dispense interface to a drug delivery device.

Further to this, the lockout element may be configured such that when said dispense interface is attached to said drug delivery device, a distal portion of the drug delivery device acts on said bearing section such that said spring element is strained or further strained.

Accordingly, by attaching the dispense interface to the drug delivery device the energy stored in the spring element is increased. This enables the lockout element to reliably change its condition. In particular, the lockout element may thereby be moved from the receptive condition to the activated condition.

Moreover, the lockout element may be configured such that when said dispense interface is detached from said drug delivery device, a distal portion of the drug delivery device is retracted from said bearing section such that said spring element is at least partly relaxed and said bearing section is moved from the open to the blocking position.

Thus, the energy stored in the spring element in the receptive and/or activated condition may in a simple and reliable manner effect the lockout element to move to the locked condition, in which the bearing section is in its blocking position. At the same time the detachment of the dispense interface may be supported by the relaxing process of the spring element.

In a further preferred embodiment, the lockout element may have at least a release section with a shaped element, wherein the inner body has at least a retaining element, and wherein in the receptive condition the shaped element is in releasable engagement with the retaining element. Accordingly, the shaped element is in disengagement with the retaining element in the activated and/or the locked condition.

Hereby, the shaped element corresponds to the retaining element, wherein the releasable engagement may preferably be configured as a positive fit. In particular, the shaped element may be formed as a recess and the retaining element may be formed as a protrusion and wherein in the releasable engagement the protrusion at least partly. protrudes in the recess.

By providing a lockout element with a release section, which comprises a shaped element, and an inner body with a retaining element, which corresponds to the shaped element, the lockout element may securely be held in the receptive condition. In particular, this enables to securely hold the spring element strained as long as the lockout element is in a receptive condition. This prevents the lockout element from being moved from the receptive to the locked condition unintentionally. Thus, it is thereby ensured that the dispense interface remains attachable as long as it has not been used.

It is furthermore preferred, that the lockout element is configured such that when said dispense interface is attached to said drug delivery device, a distal portion of the drug delivery device acts on said bearing section such that said shaped element is released out of engagement with said retaining element.

Thus, by attaching the dispense interface to the drug delivery device the shaped element may reliably released out of engagement with the retaining element. In this released condition the lockout element is in its activated condition, in which it moves automatically to the locked condition when the dispense interface is detached from the drug delivery device.

Hence, in the released condition the spring element is no longer retained in its strained condition by the positive engagement of the shaped element and the retaining element. However, the spring element is further held in its strained condition by the distal portion of the drug delivery device acting on the bearing portion as long as the dispense interface is attached to the drug delivery device. As soon as the dispense interface is detached from the drug delivery device, the distal portion of the drug delivery device is retracted from the bearing portion, thus allowing the spring element, which is no longer held by the release section, to relax.

Furthermore, in the engagement condition of the shaped element and the retaining element, the release section of the lockout element may be in a strained condition. Consequently, by releasing the shaped element out of engagement with the retaining element, the release section may relax, thus maintaining or securing the released condition.

It is moreover preferred, that the lockout element is attached to the inner body by a connecting element. The attachment of the lockout element to the inner body may thereby be safely maintained particularly in the locked condition. This prevents an inadvertent removal of the lockout element from the inner body and accordingly further reduces the risk of reattachment of the dispense interface after it has been used.

The connecting element may be formed as an edge section, which is in engagement with a corresponding surface section of the inner body. Likewise the connection section may comprise a recess, through which a protrusion of the inner body at least partially protrudes. According to a further embodiment, the connection section may be formed by a connecting portion, which is engaged with an undercut of the inner body. In any of the mentioned embodiments of the connecting element, an at least partly positive fit with the inner body may be provided.

The dispense interface may be produced cost effectively, in case the lockout element is formed as one piece. Preferably the lockout element may be formed from metal, particularly from a flat metal. Likewise the lockout element may be formed from plastic material, particularly from a flat plastic material.

According to a further embodiment, the spring element and the release section adjoin the bearing section independently. This allows the spring element and the release section to be designed independently, whereby optimal use properties of the lockout element may be achieved.

According to another embodiment, the release section adjoins the spring element and the bearing section is provided on the spring element. This embodiment allows the lockout element to be designed in a particularly simple manner and therefore be manufactured cost-effectively.

It is especially advantageous if the support section is provided on the release section, whereby likewise a simple constructive design and therefore a cost-effective manufacturability may be ensured.

The dispense interface is preferably configured to be used with a drug delivery device, in particular with a drug delivery device mentioned at the beginning, whereby the dispense interface is removably attached to the drug delivery device. By detaching the dispense interface from the drug delivery device, it may, due to the lockout element moving to the locked condition, not be reattached to the drug delivery device. The risk of contamination from residual drug components within the dispense interface is thus eliminated.

These as well as other advantages of various aspects of the present invention will become apparent to those of ordinary skill in the art by reading the following detailed description, with appropriate reference to the accompanying drawings, in which:
Fig. 1 illustrates a perspective view of a delivery device with an end cap of the device removed;
Fig. 2 illustrates a perspective view of the delivery device distal end showing the cartridge;
Fig. 3 illustrates a perspective view of the delivery device illustrated in Fig. 1 or 2 with one cartridge retainer in an open position;
Fig. 4 illustrates a dispense interface and a dose dispenser that may be removably mounted on a distal end of the delivery device illustrated in Fig. 1;
Fig. 5 illustrates the dispense interface and the dose dispenser illustrated in Fig. 4 mounted on a distal end of the delivery device illustrated in Fig. 1;
Fig. 6 illustrates one arrangement of a needle assembly that may be mounted on a distal end of the delivery device;
Fig. 7 illustrates a perspective view of the dispense interface illustrated in Fig. 4;
Fig. 8 illustrates another perspective view of the dispense interface illustrated in Fig. 4;
Fig. 9 illustrates a cross-sectional view of the dispense interface illustrated in Fig. 4;
Fig. 10 illustrates an exploded view of the dispense interface illustrated in Fig. 4;
Fig. 11 illustrates a cross-sectional view of the dispense interface and needle assembly mounted onto a drug delivery device, such as the device illustrated in Fig. 1;
Fig. 12 illustrates a perspective view of the dispense interface with an inner body and a lockout element;
Fig. 13 illustrates an exploded view of the dispense interface illustrated in Fig. 12;
Fig. 14 illustrates a perspective view of a lockout element according to a first embodiment;
Fig. 15 illustrates a side view of a lockout element according to a first embodiment in a receptive condition coupled to an inner body of the dispense interface;
Fig. 16 illustrates a side view of a lockout element according to a first embodiment in a locked condition coupled to an inner body of the dispense interface;
Fig. 17 illustrates a perspective view of a lockout element according to a second embodiment in a receptive condition;
Fig. 18 illustrates a side view of a lockout element according to a second embodiment in a receptive condition coupled to an inner body of the dispense interface;
Fig. 19 illustrates a perspective view of a lockout element according to a second embodiment in an activated condition;
Fig. 20 illustrates a side view of a lockout element according to a second embodiment in an activated condition coupled to an inner body of the dispense interface;
Fig. 21 illustrates a perspective view of a lockout element according to a second embodiment in a locked condition;
Fig. 22 illustrates a side view of a lockout element according to a second embodiment in a locked condition coupled to an inner body of the dispense interface;
Fig. 23 illustrates a perspective view of a lockout element according to a third embodiment in a receptive condition coupled to an inner body of the dispense interface;
Fig. 24 illustrates a side view of a lockout element according to a third embodiment in a receptive condition coupled to an inner body of the dispense interface;
Fig. 25 illustrates a perspective view of a lockout element according to a third embodiment in an activated condition coupled to an inner body of the dispense interface;
Fig. 26 illustrates a side view of a lockout element according to a third embodiment in an activated condition coupled to an inner body of the dispense interface;
Fig. 27 illustrates a perspective view of a lockout element according to a third embodiment in a locked condition coupled to an inner body of the dispense interface;
Fig. 28 illustrates a side view of a lockout element according to a third embodiment in a locked condition coupled to an inner body of the dispense interface.

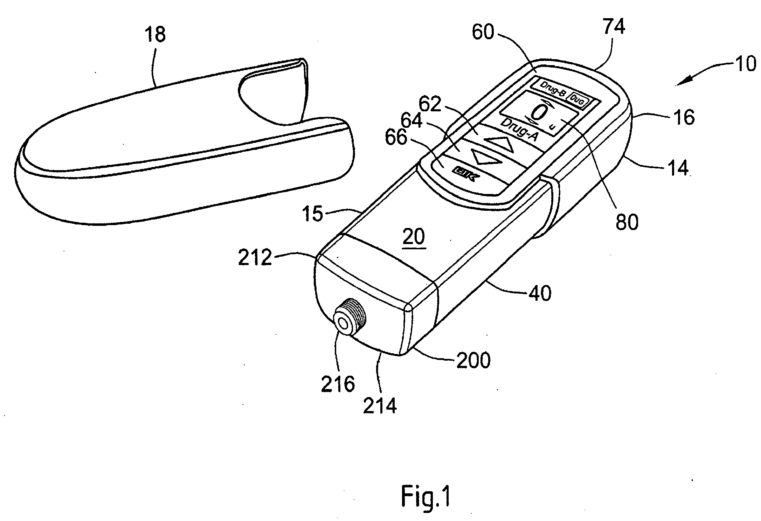

The drug delivery device illustrated in Fig. 1 comprises a main body 14 that extends from a proximal end 16 to a distal end 15. At the distal end 15, a removable end cap or cover 18 is provided. This end cap 18 and the distal end 15 of the main body 14 work together to provide a snap fit or form fit connection so that once the cover 18 is slid onto the distal end 15 of the main body 14, this frictional fit between the cap and the main body outer surface 20 prevents the cover from inadvertently falling off the main body.

The main body 14 contains a micro-processor control unit, an electro-mechanical drive train, and at least two medicament reservoirs. When the end cap or cover 18 is removed from the device 10 (as illustrated in Fig. 1), a dispense interface 200 is mounted to the distal end 15 of the main body 14, and a dose dispenser (e.g., a needle assembly) is attached to the interface. The drug delivery device 10 can be used to administer a computed dose of a second medicament (secondary drug compound) and a variable dose of a first medicament (primary drug compound) through a single needle assembly, such as a double ended needle assembly.

The drive train may exert a pressure on the bung of each cartridge, respectively, in order to expel the doses of the first and second medicaments. For example, a piston rod may push the bung of a cartridge forward a pre-determined amount for a single dose of medicament. When the cartridge is empty, the piston rod is retracted completely inside the main body 14, so that the empty cartridge can be removed and a new cartridge can be inserted.

A control panel region 60 is provided near the proximal end of the main body 14. Preferably, this control panel region 60 comprises a digital display 80 along with a plurality of human interface elements that can be manipulated by a user to set and inject a combined dose. In this arrangement, the control panel region comprises a first dose setting button 62, a second dose setting button 64 and a third button 66 designated with the symbol "OK." In addition, along the most proximal end of the main body, an injection button 74 is also provided (not visible in the perspective view of Fig. 1).

The cartridge holder 40 can be removably attached to the main body 14 and may contain at least two cartridge retainers 50 and 52. Each retainer is configured so as to contain one medicament reservoir, such as a glass cartridge. Preferably, each cartridge contains a different medicament.

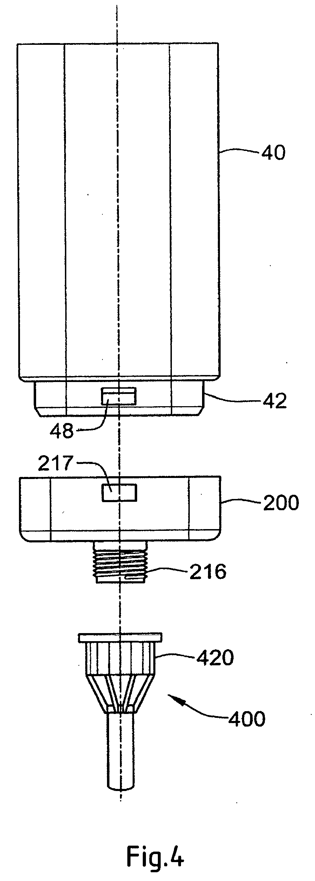

In addition, at the distal end of the cartridge holder 40, the drug delivery device illustrated in Fig. 1 includes a dispense interface 200. As will be described in relation to Fig. 4, in one arrangement, this dispense interface 200 includes a main outer body 212 that is removably attached to a distal end 42 of the cartridge housing 40. As can be seen in Fig. 1, a distal end 214 of the dispense interface 200 preferably comprises a needle hub 216. This needle hub 216 may be configured so as to allow a dose dispenser, such as a conventional pen type injection needle assembly, to be removably mounted to the drug delivery device 10.

Once the device is turned on, the digital display 80 shown in Fig. 1 illuminates and provides the user certain device information, preferably information relating to the medicaments contained within the cartridge holder 40. For example, the user is provided with certain information relating to both the primary medicament (Drug A) and the secondary medicament (Drug B).

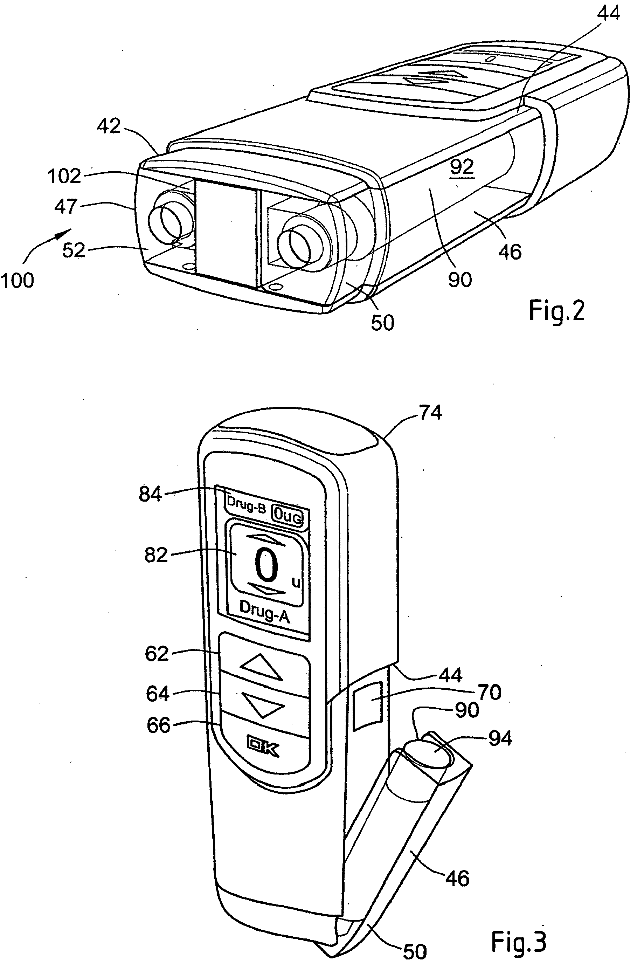

As shown in Fig. 3, the first and second cartridge retainers 50, 52 may be hinged cartridge retainers. These hinged retainers allow user access to the cartridges. Fig. 3 illustrates a perspective view of the cartridge holder 40 illustrated in Fig. 1 with the first hinged cartridge retainer 50 in an open position. Fig. 3 illustrates how a user might access the first cartridge 90 by opening up the first retainer 50 and thereby having access to the first cartridge 90.

As mentioned above when discussing Fig. 1, a dispense interface 200 is coupled to the distal end of the cartridge holder 40. Fig. 4 illustrates a flat view of the dispense interface 200 unconnected to the distal end of the cartridge holder 40. A dose dispenser or needle assembly that may be used with the interface 200 is also illustrated and is provided in a protective outer cap 420.

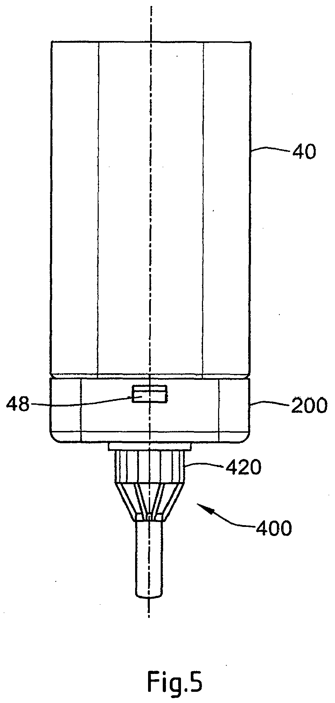

In Fig. 5, the dispense interface 200 illustrated in Fig. 4 is shown coupled to the cartridge holder 40. The axial attachment means between the dispense interface 200 and the cartridge holder 40 can be any known axial attachment means to those skilled in the art, including snap locks, snap fits, snap rings, keyed slots, and combinations of such connections. The connection or attachment between the dispense interface and the cartridge holder may also contain additional features (not shown), such as connectors, stops, splines, ribs, grooves, pips, clips and the like design features, that ensure that specific hubs are attachable only to matching drug delivery devices. Such additional features would prevent the insertion of a non-appropriate secondary cartridge to a non-matching injection device.

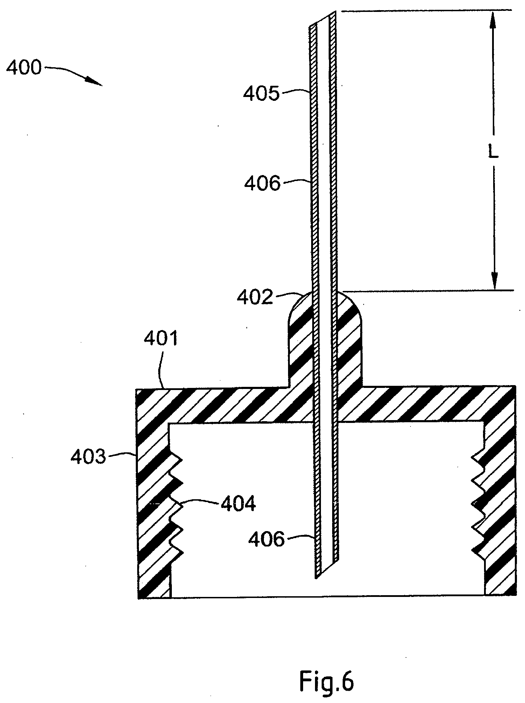

Fig. 5 also illustrates the needle assembly 400 and protective cover 420 coupled to the distal end of the dispense interface 200 that may be screwed onto the needle hub of the interface 200. Fig. 6 illustrates a cross sectional view of the double ended needle assembly 402 mounted on the dispense interface 200 in Fig. 5.

The needle assembly 400 illustrated in Fig. 6 comprises a double ended needle 406 and a hub 401. The double ended needle or cannula 406 is fixedly mounted in a needle hub 401. This needle hub 401 comprises a circular disk shaped element which has along its periphery a circumferential depending sleeve 403. Along an inner wall of this hub member 401, a thread 404 is provided. This thread 404 allows the needle hub 401 to be screwed onto the dispense interface 200 which, in one preferred arrangement, is provided with a corresponding outer thread along a distal hub. At a center portion of the hub element 401 there is provided a protrusion 402. This protrusion 402 projects from the hub in an opposite direction of the sleeve member. A double ended needle 406 is mounted centrally through the protrusion 402 and the needle hub 401. This double ended needle 406 is mounted such that a first or distal piercing end 405 of the double ended needle forms an injecting part for piercing an injection site (e.g., the skin of a user).

Similarly, a second or proximal piercing end 406 of the needle assembly 400 protrudes from an opposite side of the circular disc so that it is concentrically surrounded by the sleeve 403. In one needle assembly arrangement, the second or proximal piercing end 406 may be shorter than the sleeve 403 so that this sleeve to some extent protects the pointed end of the back sleeve. The needle cover cap 420 illustrated in Fig. 4 and 5 provides a form fit around the outer surface 403 of the hub 401.

Referring now to Fig. 4 to 11, one preferred arrangement of this interface 200 will now be discussed. In this one preferred arrangement, this interface 200 comprises:
a. a main outer body 210,
b. an first inner body 220,
c. a second inner body 230,
d. a first piercing needle 240,
e. a second piercing needle 250,
f. a valve seal 260, and
g. a septum 270.

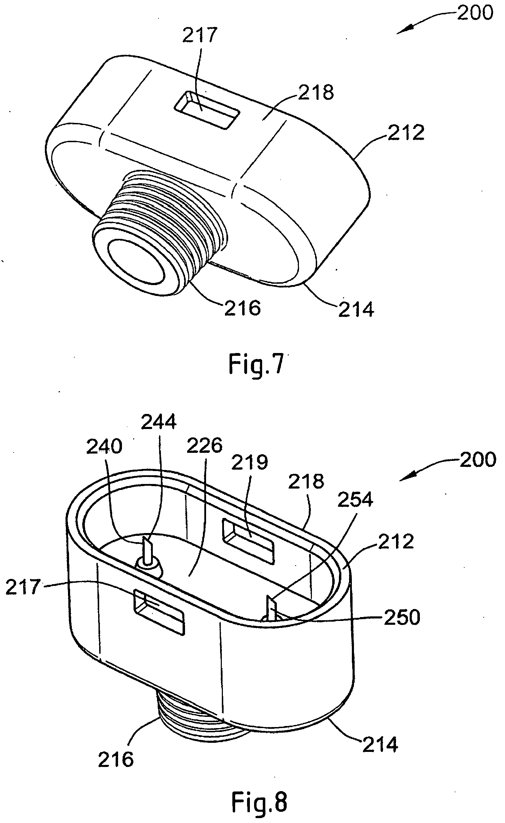

The main outer body 210 comprises a main body proximal end 212 and a main body distal end 214. At the proximal end 212 of the outer body 210, a connecting member is configured so as to allow the dispense interface 200 to be attached to the distal end of the cartridge holder 40. Preferably, the connecting member is configured so as to allow the dispense interface 200 to be removably connected the cartridge holder 40. In one preferred interface arrangement, the proximal end of the interface 200 is configured with an upwardly extending wall 218 having at least one recess. For example, as may be seen from Fig. 8, the upwardly extending wall 218 comprises at least a first recess 217 and a second recess 219.

Preferably, the first and the second recesses 217, 219 are positioned within this main outer body wall so as to cooperate with an outwardly protruding member located near the distal end of the cartridge housing 40 of the drug delivery device 10. For example, this outwardly protruding member 48 of the cartridge housing may be seen in Fig. 4 and 5. A second similar protruding member is provided on the opposite side of the cartridge housing. As such, when the interface 200 is axially slid over the distal end of the cartridge housing 40, the outwardly protruding members will cooperate with the first and second recess 217, 219 to form an interference fit, form fit, or snap lock. Alternatively, and as those of skill in the art will recognize, any other similar connection mechanism that allows for the dispense interface and the cartridge housing 40 to be axially coupled could be used as well.

The main outer body 210 and the distal end of the cartridge holder 40 act to form an axially engaging snap lock or snap fit arrangement that could be axially slid onto the distal end of the cartridge housing. In one alternative arrangement, the dispense interface 200 may be provided with a coding feature so as to prevent inadvertent dispense interface cross use. That is, the inner body of the hub could be geometrically configured so as to prevent an inadvertent cross use of one or more dispense interfaces.

A mounting hub is provided at a distal end of the main outer body 210 of the dispense interface 200. Such a mounting hub can be configured to be releasably connected to a needle assembly. As just one example, this connecting means 216 may comprise an outer thread that engages an inner thread provided along an inner wall surface of a needle hub of a needle assembly, such as the needle assembly 400 illustrated in Fig. 6. Alternative releasable connectors may also be provided such as a snap lock, a snap lock released through threads, a bayonet lock, a form fit, or other similar connection arrangements.

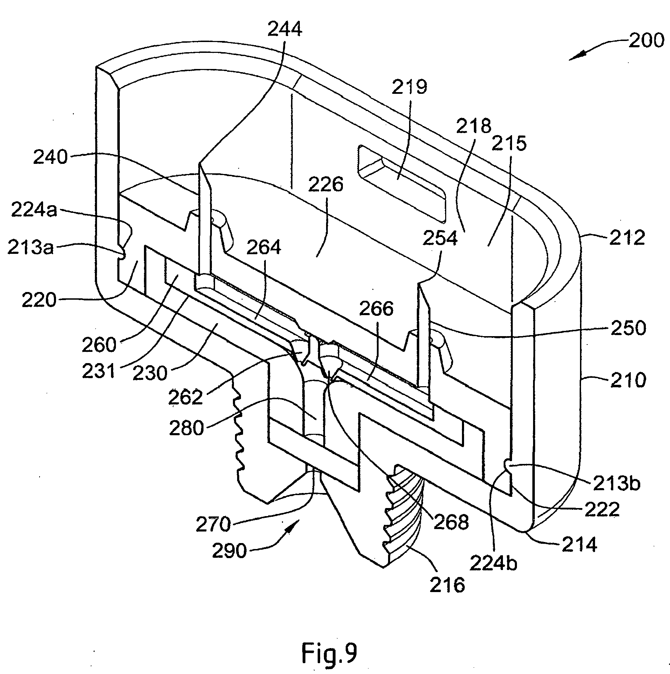

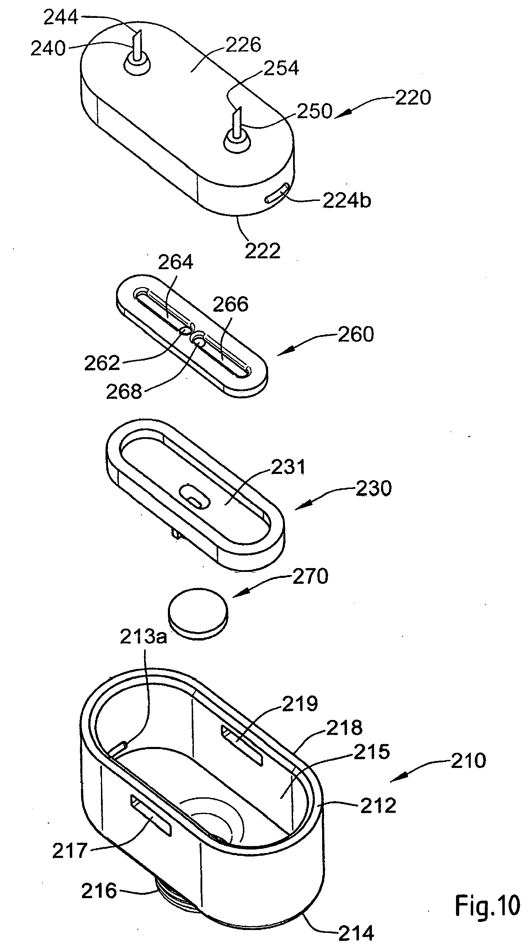

The dispense interface 200 further comprises a first inner body 220. Certain details of this inner body are illustrated in Fig. 8-11. Preferably, this first inner body 220 is coupled to an inner surface 215 of the extending wall 218 of the main outer body 210. More preferably, this first inner body 220 is coupled by way of a rib and groove form fit arrangement to an inner surface of the outer body 210. For example, as can be seen from Fig. 9, the extending wall 218 of the main outer body 210 is provided with a first rib 213a and a second rib 213b. This first rib 213a is also illustrated in Fig. 10. These ribs 213a and 213b are positioned along the inner surface 215 of the wall 218 of the outer body 210 and create a form fit or snap lock engagement with cooperating grooves 224a and 224b of the first inner body 220. In a preferred arrangement, these cooperating grooves 224a and 224b are provided along an outer surface 222 of the first inner body 220.

In addition, as can be seen in Fig. 8-10, a proximal surface 226 near the proximal end of the first inner body 220 may be configured with at least a first proximally positioned piercing needle 240 comprising a proximal piercing end portion 244. Similarly, the first inner body 220 is configured with a second proximally positioned piercing needle 250 comprising a proximally piercing end portion 254. Both the first and second needles 240, 250 are rigidly mounted on the proximal surface 226 of the first inner body 220.

Preferably, this dispense interface 200 further comprises a valve arrangement. Such a valve arrangement could be constructed so as to prevent cross contamination of the first and second medicaments contained in the first and second reservoirs, respectively. A preferred valve arrangement may also be configured so as to prevent back flow and cross contamination of the first and second medicaments.

In one preferred system, dispense interface 200 includes a valve arrangement in the form of a valve seal 260. Such a valve seal 260 may be provided within a cavity 231 defined by the second inner body 230, so as to form a holding chamber 280. Preferably, cavity 231 resides along an upper surface of the second inner body 230. This valve seal comprises an upper surface that defines both a first fluid groove 264 and second fluid groove 266. For example, Fig. 9 illustrates the position of the valve seal 260, seated between the first inner body 220 and the second inner body 230. During an injection step, this seal valve 260 helps to prevent the primary medicament in the first pathway from migrating to the secondary medicament in the second pathway, while also preventing the secondary medicament in the second pathway from migrating to the primary medicament in the first pathway. Preferably, this seal valve 260 comprises a first non-return valve 262 and a second non-return valve 268. As such, the first non-return valve 262 prevents fluid transferring along the first fluid pathway 264, for example a groove in the seal valve 260, from returning back into this pathway 264. Similarly, the second non-return valve 268 prevents fluid transferring along the second fluid pathway 266 from returning back into this pathway 266.

Together, the first and second grooves 264, 266 converge towards the non-return valves 262 and 268 respectively, to then provide for an output fluid path or a holding chamber 280. This holding chamber 280 is defined by an inner chamber defined by a distal end of the second inner body both the first and the second non return valves 262, 268 along with a pierceable septum 270. As illustrated, this pierceable septum 270 is positioned between a distal end portion of the second inner body 230 and an inner surface defined by the needle hub of the main outer body 210.

The holding chamber 280 terminates at an outlet port of the interface 200. This outlet port 290 is preferably centrally located in the needle hub of the interface 200 and assists in maintaining the pierceable seal 270 in a stationary position. As such, when a double ended needle assembly is attached to the needle hub of the interface (such as the double ended needle illustrated in Fig. 6), the output fluid path allows both medicaments to be in fluid communication with the attached needle assembly.

The hub interface 200 further comprises a second inner body 230. As can be seen from Fig. 9, this second inner body 230 has an upper surface that defines a recess, and the valve seal 260 is positioned within this recess. Therefore, when the interface 200 is assembled as shown in Fig. 9, the second inner body 230 will be positioned between a distal end of the outer body 210 and the first inner body 220. Together, second inner body 230 and the main outer body hold the septum 270 in place. The distal end of the inner body 230 may also form a cavity or holding chamber that can be configured to be fluid communication with both the first groove 264 and the second groove 266 of the valve seal.

Axially sliding the main outer body 210 over the distal end of the drug delivery device attaches the dispense interface 200 to the multi-use device. In this manner, a fluid communication may be created between the first needle 240 and the second needle 250 with the primary medicament of the first cartridge and the secondary medicament of the second cartridge, respectively.

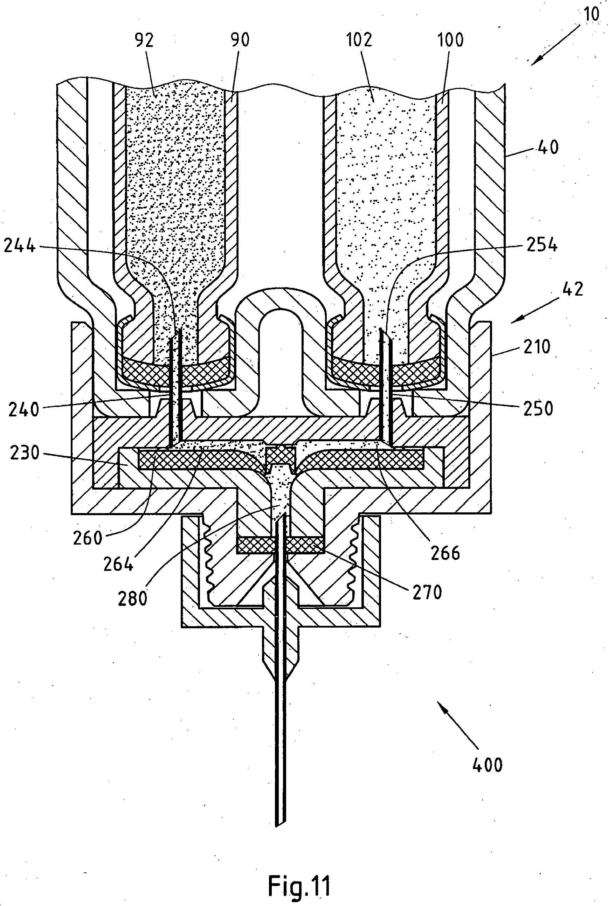

Fig. 11 illustrates the dispense interface 200 after it has been mounted onto the distal end 42 of the cartridge holder 40 of the drug delivery device 10 illustrated in Fig. 1. A double ended needle 400 is also mounted to the distal end of this interface. The cartridge holder 40 is illustrated as having a first cartridge containing a first medicament and a second cartridge containing a second medicament.

When the interface 200 is first mounted over the distal end of the cartridge holder 40, the proximal piercing end 244 of the first piercing needle 240 pierces the septum of the first cartridge 90 and thereby resides in fluid communication with the primary medicament 92 of the first cartridge 90. A distal end of the first piercing needle 240 will also be in fluid communication with a first fluid path groove 264 defined by the valve seal 260.

Similarly, the proximal piercing end 254 of the second piercing needle 250 pierces the septum of the second cartridge 100 and thereby resides in fluid communication with the secondary medicament 102 of the second cartridge 100. A distal end of this second piercing needle 250 will also be in fluid communication with a second fluid path groove 266 defined by the valve seal 260.

Fig. 11 illustrates a preferred arrangement of such a dispense interface 200 that is coupled to a distal end 15 of the main body 14 of drug delivery device 10. Preferably, such a dispense interface 200 is removably coupled to the cartridge holder 40 of the drug delivery device 10.

As illustrated in Fig. 11, the dispense interface 200 is coupled to the distal end of a cartridge housing 40. This cartridge holder 40 is illustrated as containing the first cartridge 90 containing the primary medicament 92 and the second cartridge 100 containing the secondary medicament 102. Once coupled to the cartridge housing 40, the dispense interface 200 essentially provides a mechanism for providing a fluid communication path from the first and second cartridges 90, 100 to the common holding chamber 280. This holding chamber 280 is illustrated as being in fluid communication with a dose dispenser. Here, as illustrated, this dose dispenser comprises the double ended needle assembly 400. As illustrated, the proximal end of the double ended needle assembly is in fluid communication with the chamber 280.

In one preferred arrangement, the dispense interface is configured so that it attaches to the main body in only one orientation, that is it is fitted only one way round. As such as illustrated in Fig. 11, once the dispense interface 200 is attached to the cartridge holder 40, the primary needle 240 can only be used for fluid communication with the primary medicament 92 of the first cartridge 90 and the interface 200 would be prevented from being reattached to the holder 40 so that the primary needle 240 could now be used for fluid communication with the secondary medicament 102 of the second cartridge 100. Such a one way around connecting mechanism may help to reduce potential cross contamination between the two medicaments 92 and 102.

Embodiments of a dispense interface with a lockout element and an inner body will be described in detail hereinafter.

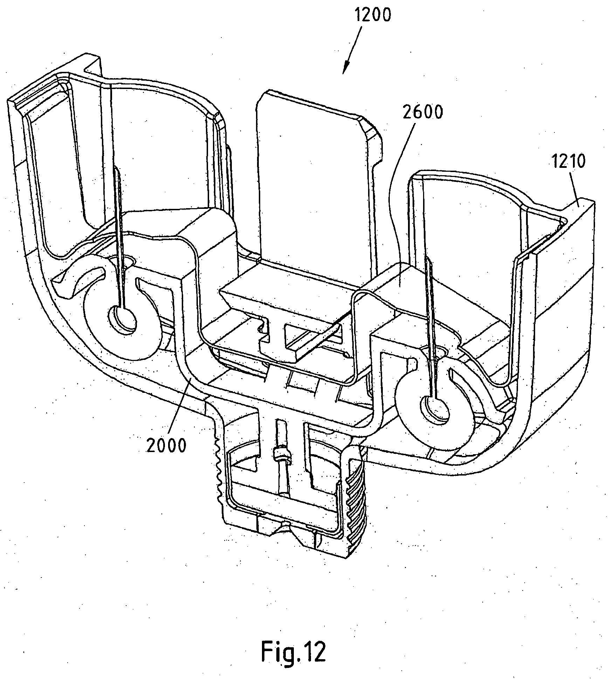

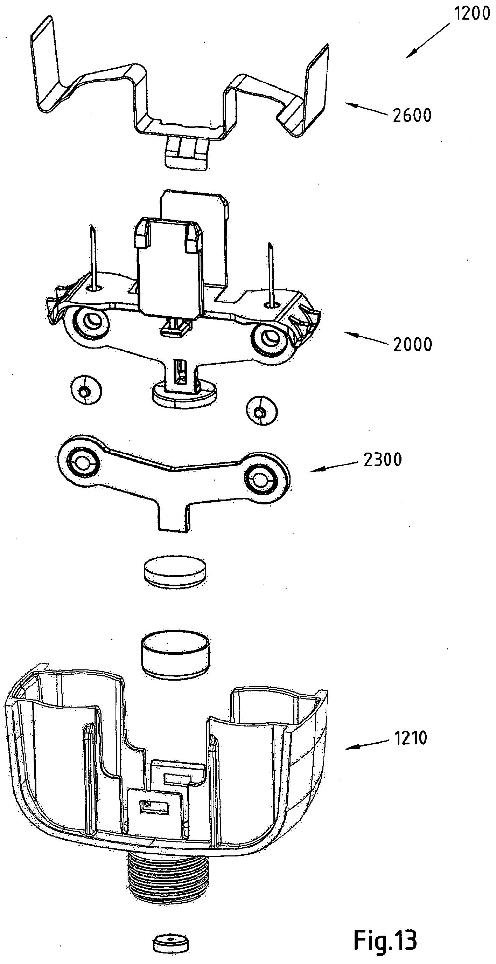

Fig. 12 and 13 show a dispense interface 1200. As may be seen from Fig. 12 and the exploded view in Fig. 13, the dispense interface 1200 may comprise an outer body 1210 and in inner body 2000. The inner body 2000 may be seated within an interior space defined by the outer main body 1210. Preferably, it is the inner body 2000 of the dispense interface 1200 that is configured to be coupled to a distal end of a drug delivery device while also being securely positioned within an interior space defined by the outer body 1210. The dispense interface 1200 may further comprise a manifold 2300.

As may be further be seen from Fig. 12 and 13 the dispense interface 2000 also comprises a lockout element in the form of a lockout spring 2600. One reason that a lockout element 2600 may be incorporated into a dispense interface 1200, is to ensure that once the dispense interface 1200 is removed from the drug delivery device, the dispense interface 1200 cannot be re-attached and used a second time. Preventing reattachment tends to ensure that medicament is not allowed to reside in the dispense interface 1200 indefinitely and contaminate the drug delivered to the patient.

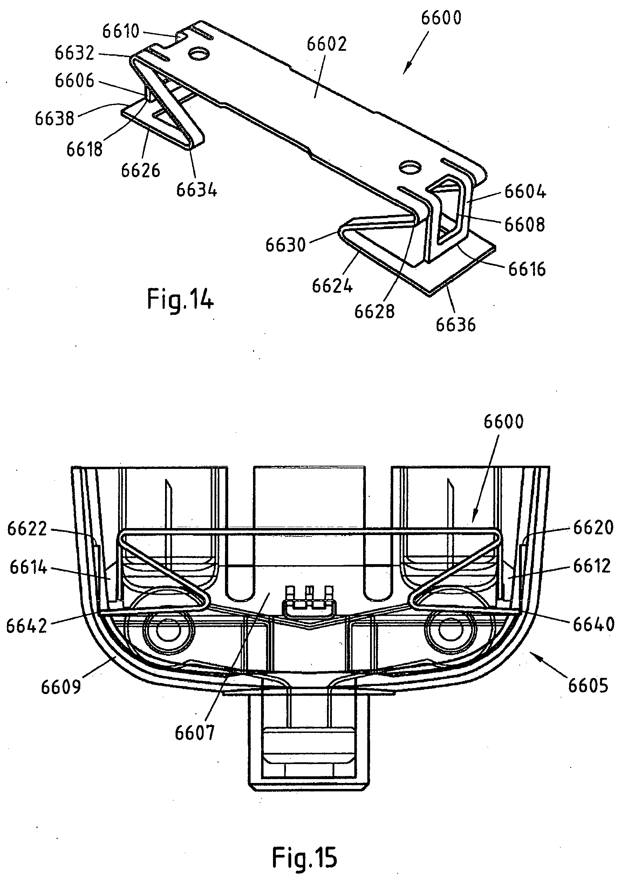

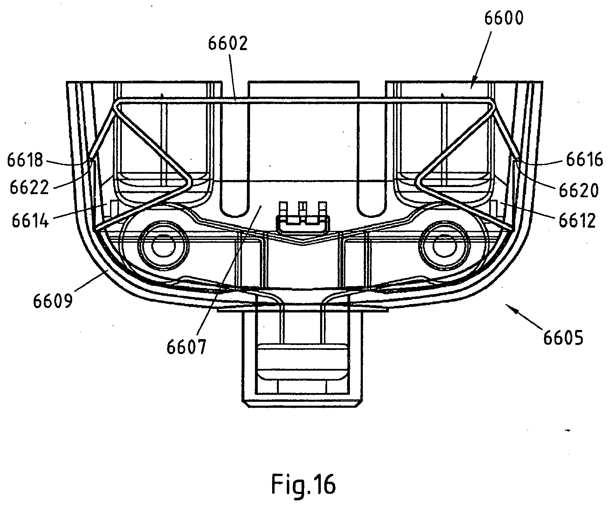

Fig. 14 shows a first embodiment of a lockout element 6600 according to the invention, and Fig. 15 and 16 show and a dispense interface 6605 comprising a lockout element 6600 according to the invention.

Specifically, Fig. 14 illustrates a perspective view of a lockout element 6600 in the form of a platform spring. The lockout element 6600 is formed as one piece from a flexible material such as a suitable plastic material or a suitable metal material. The lockout element 6600 comprises a bearing section 6602 in the form of a platform, which provides the lockout functionality of the lockout element 6600. Thus the bearing section 6602 in the form of a platform is configured to bear a distal portion of a drug delivery device, to which the dispense interface 6605 may be attached.

Further to this, the lockout element 6600 comprises release sections 6604 and 6606, which adjoin the bearing section 6602 on opposite sides. Thereby, shaped elements 6608 and 6610 are formed on the release sections 6604 and 6606. The shaped elements 6608 and 6610 are formed as recesses, which may be engaged with corresponding retaining elements 6612 and 6614 of the inner body 6607. The retaining elements 6612 and 6614 may be formed as protrusions as shown in Fig. 15 and 16.

At the free end of the release sections 6604 and 6606 support sections 6616 and 6618 are provided, which may engage support surfaces 6620 and 6622 of the outer body 6609, as illustrated in Fig. 16.

The lockout element 6600 also comprises two spring elements 6624 and 6626, which adjoin the bearing section 6602 on opposite sides of the lockout element 6600 independent of the release sections 6604 and 6606. The spring elements 6624 and 6626 are formed as spring arms with each at least one curved section. Here, the spring element 6626 has a first curved section 6628 and a second curved section 6630 and the spring element 6618 has a first curved section 6632 and a second curved section 6634. At the free ends of the spring elements 6624 and 6626 connecting element 6636 and 6638 are formed as edges, which engage corresponding surfaces 6640 sand 6642 of the outer body 6609. Thereby the lockout element 6600 is securely connected to the outer body 6609 of the dispense interface 6605.

Fig. 15 illustrates a dispense interface 6605 comprising a platform spring lockout element 6600 in the receptive condition. In this illustrated arrangement, the platform lockout element 6600 is held in a strained state on assembly. Thus, the spring arms 6624 and 6626 are in a strained condition and the shaped elements 6608 and 6610 of the release sections 6604 and 6606 are in engagement with the retaining elements 6612 and 6614. Further, the bearing section 6602 is in an open position, in which the dispense interface 6605 is attachable to a drug delivery device.

Upon fitting the dispense interface 6605 to the distal end of a drug delivery device, a distal portion of the drug delivery device acts on the bearing section 6602, whereby the shaped elements 6608 and 6610 are released out of engagement with the retaining elements 6612 and 6614. The lockout element 6600 is thus moved into an activated condition. In this activated condition the spring elements 6624 and 6626 remain strained by the distal portion of the drug delivery device acting on the bearing section 6602 as long as the dispense interface remains attached to the drug delivery device.

When the dispense interface 6605 is detached from the drug delivery device, the distal portion of the drug delivery device is retracted from the bearing section 6602. Since in this condition the shaped elements 6608 and 6610 are released out of engagement with the retaining elements 6612 and 6614, the spring elements 6624 and 6626 are enabled to relax. The lockout element 6600 is thereby moved into the locked condition, which is illustrated in Fig. 16.

In the locked condition of the lockout element 6600 the bearing section 6602 is moved to a blocking position, in which the release sections 6604 and 6606 with their support sections 6616 and 6618 are in engagement with support surfaces 6620 and 6622. By this engagement the bearing section 6602 is prevented from being moved from the blocking position back into the open position, thus the lockout element 6600 is prevented from being moved back to the receptive condition. This ensures that the dispense interface 6605 is not reattached to a drug delivery device after it has been used.

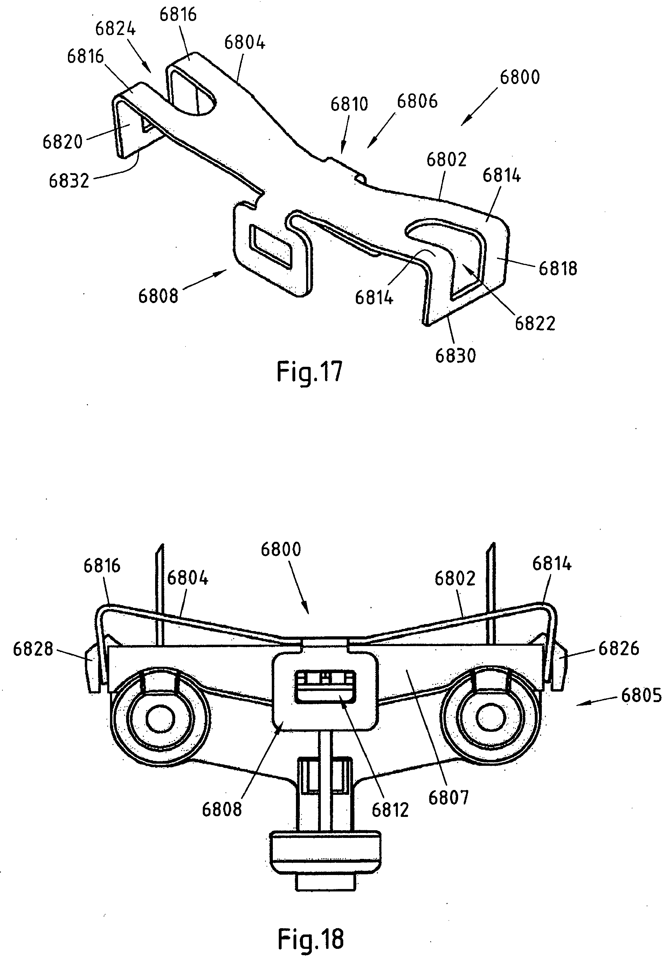

Fig. 17, 19 and 21 show a second embodiment of a lockout element 6800 according to the invention, and Fig. 18, 20 and 22 show a dispense interface 6805 comprising a lockout element 6800 according to the invention.

As illustrated in Fig. 17, the lockout element 6800 is formed as a lockout spring as one piece from a flexible material such as a suitable plastic material or a suitable metal material.

The lockout element 6800 comprises spring elements 6802 and 6804, which are formed as spring arms and which are pivotably positioned near to a center portion 6806 of the locking member 6800. The center portion 6806 of the locking member 6800 further comprises connecting elements 6808 and 6810 in the form of recesses, which engage a non-return clip or a protrusion 6812 provided along the external surface of the inner body 6807 of the dispense interface 6805, as illustrated in Fig. 18. Thereby the lockout element 6800 is securely connected to the inner body 6807.

The spring elements 6802 and 6804 further comprise bearing sections 6814 and 6816 for bearing a distal portion of a drug delivery device. Thus the bearing sections 6814 and 6816 provide the lockout functionality of the lockout element 6800.

Furthermore, the lockout element 6800 comprises release sections 6818 and 6820, which adjoin the bearing sections 6814 and 6816. Thereby, shaped elements 6822 and 6824 are formed on the release sections 6818 and 6820. The shaped elements 6822 and 6824 are formed as recesses, which may be engaged with corresponding retaining elements 6826 and 6828 of the inner body 6807, whereas the retaining elements 6826 and 6828 may be formed as protrusions as shown in Fig. 18, 20 and 22.

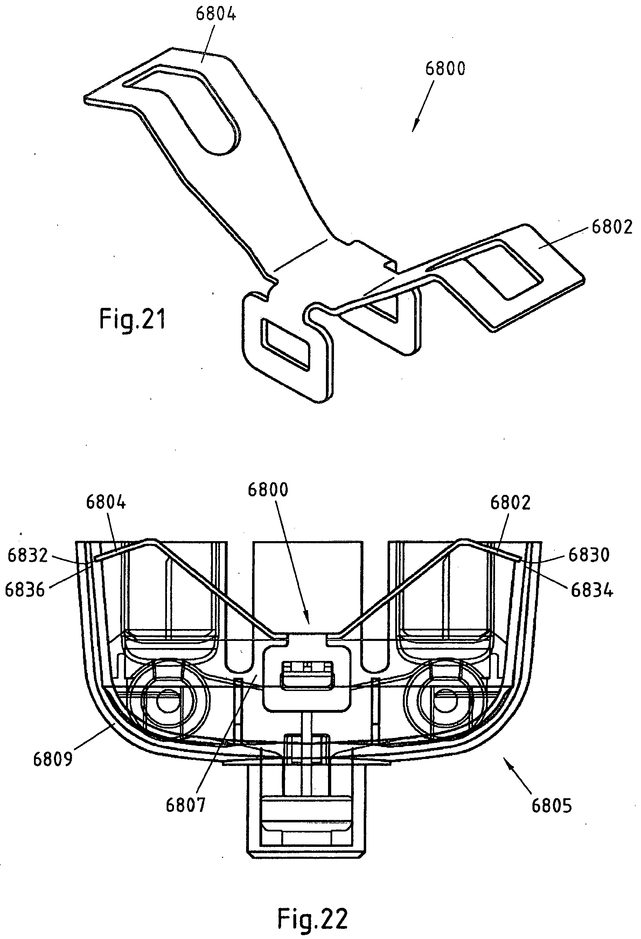

At the free end of the release sections 6818 and 6820 support sections 6830 and 6832 are provided, which may engage support surfaces 6834 and 6836 of the outer body 6809, as illustrated in Fig. 22.

Fig. 17 and 18 illustrate the lockout element 6800 in a receptive condition, in which the dispense interface 6805 is attachable to a drug delivery device. As can be seen from Fig. 18 particularly, in the receptive condition, the spring elements 6802 and 6804 of the locking element 6800 reside slightly above the top surface of the inner body 6807 with the connecting element 6808 engaged with the non-return clip 6812. In this position, the spring elements 6802 and 6804 are strained in a proximal direction and the bearing sections 6814 and 6816 are in their open position. However, the shaped elements 6822 and 6824 of the release sections 6818 and 6820 are engaged with the retaining elements 6826 and 6828 of the inner body 6807 and thereby prevent the spring elements to relax. Thus, the bearing sections are also prevented from moving to the blocking position.

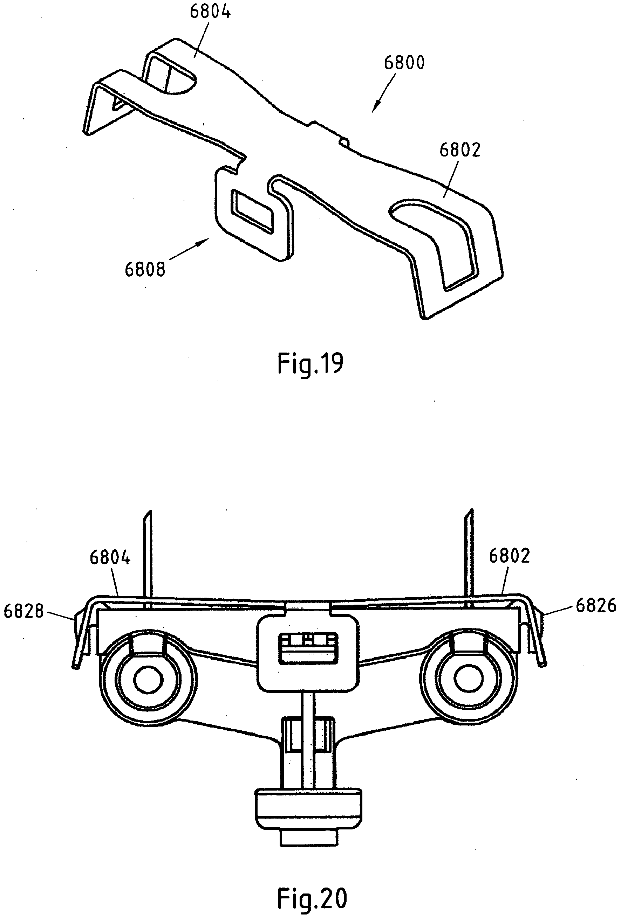

Fig. 19 and 20 illustrate a side view of locking member 6800 within the dispense interface 6805 in the activated condition, wherein Fig. 20 shows the locking member 6800 connected to the inner body 6807. To activate the locking member 6800, the distal end of the drug delivery device is inserted into the dispense interface 6805 and a distal portion of the drug delivery device will act on the bearing sections 6814 and 6816 provided on the spring elements 6802 and 6804. Thereby the shaped elements 6822 and 6824 are released out of engagement with the retaining elements 6626 and 6628. The lockout element 6800 is thus moved into an activated condition. In this activated condition the spring elements 6802 and 6804 remain strained by the distal portion of the drug delivery device acting on the bearing sections 6814 and 6816 as long as the dispense interface remains attached to the drug delivery device.

Fig. 21 illustrates a perspective view of the lockout element 6800 in a locked condition and Fig. 22 illustrates this lockout element 6800 within the dispense interface 6805 in the locked condition. The lockout element 6800 moves from the activated to the locked condition when the dispense interface 6805 is detached from the drug delivery device. Thereby the distal portion of the drug delivery device is retracted from the bearing sections 6814 and 6816. Since in this condition the shaped elements 6822 and 6824 are released out of engagement with the retaining elements 6826 and 6828, the spring elements 6802 and 6804 are enabled to relax. The lockout element 6800 is thereby moved into the locked condition, which is illustrated in Fig. 22.

In the locked condition of the lockout element 6800 the bearing sections 6814 and 6816 are moved to a blocking position, in which the support sections 6830 and 6832 are in engagement with support surfaces 6834 and 6836 of the outer body 6809. By this engagement the bearing sections 6814 and 6816 are prevented from being moved from the blocking position back into the open position, thus the lockout element 6800 is prevented from being moved back to the receptive condition. This ensures that the dispense interface 6805 is not reattached to a drug delivery device after it has been used.

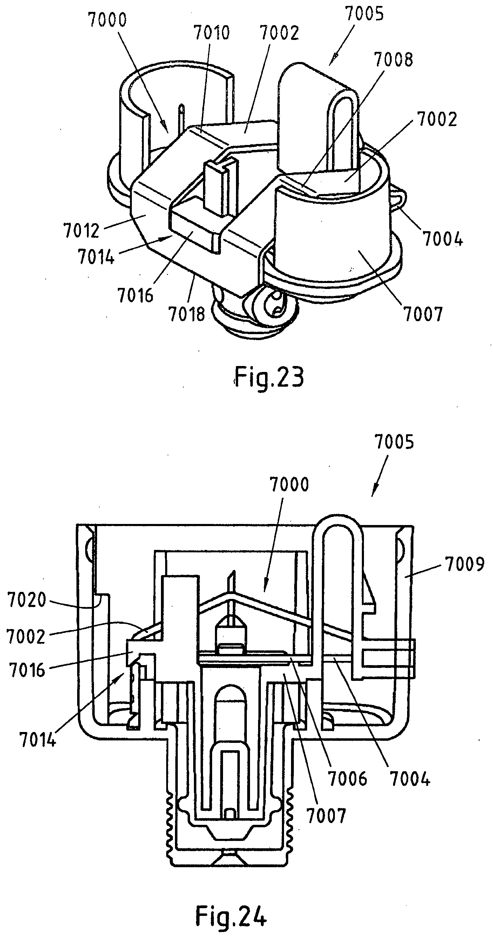

Fig. 23 to 28 show a third embodiment of a dispense interface 7005 comprising a lockout element 7000 according to the invention.

As illustrated in the perspective views in Fig. 23, 25 and 27, the lockout element 7000 is formed as a lockout spring as one piece from a flexible material such as a suitable plastic material or a suitable metal material.

The lockout element 7000 comprises a spring element 7002, which is formed as a spring arm and is pivotably positioned near a center portion 7004 of the locking member 7000. The center portion 7004 of the locking member 7000 further comprises a connecting element 7006 in the form of a material portion, which is engaged in an undercut provided on the inner body 7007 of the dispense interface 7005, as illustrated in Fig. 24, 26 and 28. Thereby the lockout element 7000 is securely connected to the inner body 7007.

The spring element 7002 further comprises bearing sections 7008 and 7010 for bearing a distal portion of a drug delivery device. Thus, the bearing sections 7008 and 7010 provide the lockout functionality of the lockout element 7000.

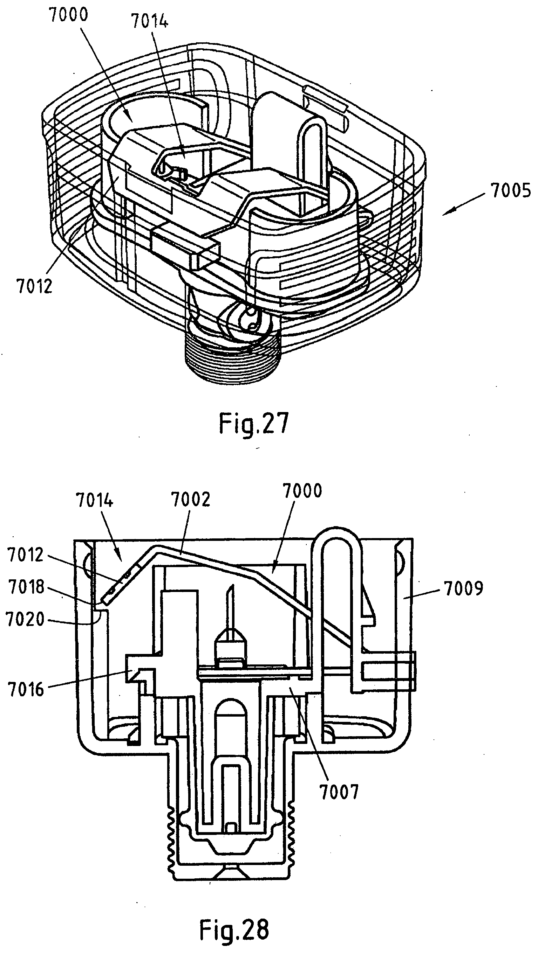

Furthermore, the lockout element 7000 comprises a release section 7012, which adjoins the spring element 7002. Thereby, a shaped element 7014 is formed on the release section 7012. The shaped element 7014 is formed as a recess, which may be engaged with a corresponding retaining element 7016 of the inner body 7007, whereas the retaining element 7016 may be formed as a protrusion as shown in Fig. 23 to 28. At the free end of the release section 7014 a support section 7018 is provided, which may engage a support surface 7020 of the outer body 7009, as illustrated in Fig. 28.

Fig. 23 and 24 illustrate the lockout element 7000 in a receptive condition, in which the dispense interface 7005 is attachable to a drug delivery device. As can be seen from Fig. 24 particularly, in the receptive condition, the spring element 7002 of the locking element 7000 resides slightly above the center portion 7006 with the connecting element 7006 engaged with an undercut of the inner body 7007.

In this position, the spring element 7002 is strained in a proximal direction and the bearing sections 7008 and 7010 are in their open position. However, the shaped element 7014 of the release section 7012 is engaged with the retaining element 7016 of the inner body 7007 and thereby prevents the spring element 7002 to relax. Thus, the bearing sections 7008 and 7010 are also prevented from moving to the blocking position.

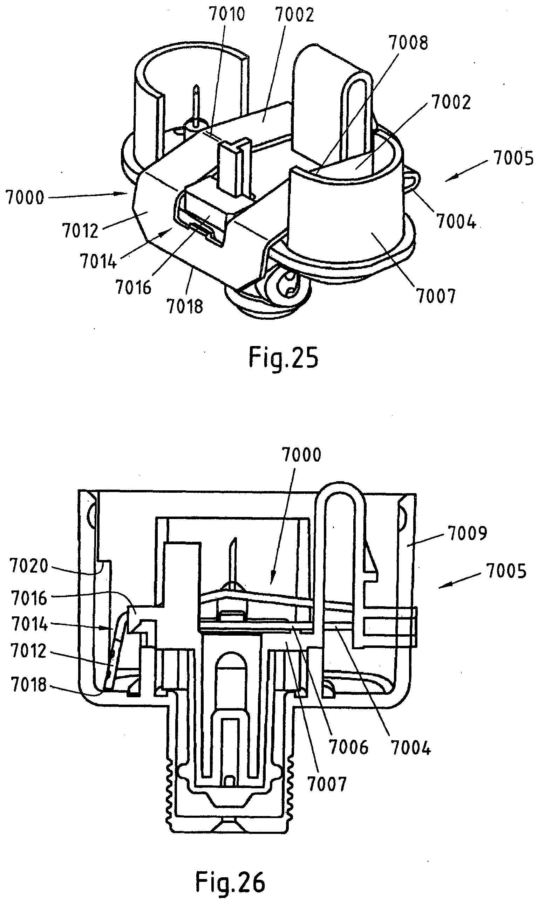

Fig. 25 and 26 illustrate the locking member 7000 within the dispense interface 7005 in the activated condition. To activate this locking member 7000, the distal end of the drug delivery device is inserted into the dispense interface 7005 and a distal portion of the drug delivery device will act on the bearing sections 7008 and 7010 provided on the spring element 7002. Thereby the shaped element 7014 is released out of engagement with the retaining element 7016. The lockout element 7000 is thus moved into an activated condition. In this activated condition the spring element 7002 remains strained by the distal portion of the drug delivery device acting on the bearing sections 7008 and 7010 as long as the dispense interface 7005 remains attached to the drug delivery device.

Fig. 27 and 28 illustrate the lockout element 7000 within the dispense interface 7005 in the locked condition. The lockout element 7000 moves from the activated to the locked condition when the dispense interface 7005 is detached from the drug delivery device. Thereby the distal portion of the drug delivery device is retracted from the bearing sections 7008 and 7010. Since in this condition the shaped element 7014 is released out of engagement with the retaining element 7016, the spring element 7002 is enabled to relax. The lockout element 7000 is thereby moved into the locked condition, which is illustrated in Fig. 27 and 28.

In the locked condition of the lockout element 7000 the bearing sections 7008 and 7010 are in a blocking position, in which the release section 7012 with its support section. 7018 is in engagement with support surface 7020 of the outer body 7009. By this engagement the bearing sections 7008 and 7010 are prevented from being moved from the blocking position back into the open position, thus the lockout element 7000 is prevented from being moved back to the receptive condition. This ensures that the dispense interface 7005 is not reattached to a drug delivery device after it has been used.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36-[Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25 IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2.,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25 Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2;
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion , N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

## Claims

1. Dispense interface for use with a drug delivery device comprising
- an inner body (6607, 6807, 7007) and
- a lockout element (6600, 6800, 7000),
- wherein the lockout element (6600, 6800, 7000) is coupled to the inner body (6607, 6807, 7007),
- wherein the lockout element (6600, 6800, 7000) is movable from a receptive condition to a locked condition,
- wherein in the receptive condition the dispense interface (6605, 6805, 7005) is attachable to the drug delivery device,
- wherein in the locked condition the dispense interface (6605, 6805, 7005) is riot attachable to the drug delivery device,
- wherein the lockout element (6600, 6800, 7000) is configured to move from the receptive condition to the locked condition when said dispense interface (6605, 6805, 7005) is attached to and detached from said drug delivery device, **characterized in that**
- the lockout element (6600, 6800, 7000) is movable from the receptive condition to an activated condition, and
- wherein in the activated condition the lockout element (6600, 6800, 7000) is configured to move to the locked condition when said dispense interface (6605, 6805, 7005) is detached from said drug delivery device, and
- wherein the lockout element (6600, 6800, 7000) is configured to move from the receptive condition to the activated condition when said dispense interface (6605, 6805, 7005) is attached to said drug delivery device.

2. Dispense interface according to claims 1, wherein the lockout element (6600, 6800, 7000) comprises at least a spring element (6624, 6626, 6802, 6804, 7002), which is strained in the receptive and/or the activated condition and at least partly relaxed in the locked condition.

3. Dispense interface according to any one of claims 1 and 2,
- wherein the lockout element (6600, 6800, 7000) comprises at least a bearing section (6602, 6814, 6816, 7008, 7010) configured to bear a distal portion of the drug delivery device,
- wherein in the receptive condition the bearing section (6602, 6814, 6816, 7008, 7010) is in an open position, in which it allows the distal portion of the drug delivery device to approach the inner body (6607, 6807, 7007), and
- wherein in the locked condition the bearing section (6602, 6814, 6816, 7008, 7010) is in a blocking position, in which it prevents the distal portion of the drug delivery device to approach the inner body (6607, 6807, 7007).

4. Dispense interface according to claim 3,
- wherein the lockout element (6600, 6800, 7000) comprises at least a support section (6616, 6618, 6830, 6832, 7018),
- wherein a support surface (6620, 6622, 6834, 6836, 7020) is provided on the inner body (6607, 6807, 7007) or on an outer body (6609, 6809, 7009) which is attached to the inner body (6607, 6807, 7007), and
- wherein the lockout element (6600, 6800, 7000) is configured such that in the locked condition the support section (6616, 6618, 6830, 6832, 7018) is in engagement with the support surface (6620, 6622, 6834, 6836, 7020) so as to prevent the bearing section (6602, 6814, 6816, 7008, 7010) from being moved into the open position.

5. Dispense interface according to any one of claims 2 and 3, wherein the bearing section (6602, 6814, 6816, 7008, 7010) is resiliently supported on the inner body (6607, 6807, 7007) by the spring element (6624, 6626, 6802, 6804, 7002).

6. Dispense interface according to claim 5, wherein the lockout element (6600, 6800, 7000) is configured such that when said dispense interface (6605, 6805, 7005) is attached to said drug delivery device, a distal portion of the drug delivery device acts on said bearing section (6602, 6814, 6816, 7008, 7010) such that said spring element (6624, 6626, 6802, 6804, 7002) is strained or further strained.

7. Dispense interface according to any one of claims 5 and 6, wherein the lockout element (6600, 6800, 7000) is configured such that when said dispense interface (6605, 6805, 7005) is detached from said drug delivery device, a distal portion of the drug delivery device is retracted from said bearing section (6602, 6814, 6816, 7008, 7010) such that said spring element (6624, 6626, 6802, 6804, 7002) is at least partly relaxed and said bearing section (6602, 6814, 6816, 7008, 7010) is moved from the open to the blocking position.

8. Dispense interface according to any one of claims 1 to 7,
- wherein the lockout element (6600, 6800, 7000) has at least a release section (6604, 6606, 6818, 6820, 7012) with a shaped element (6608, 6610, 6822, 6824, 7014),
- wherein the inner body (6607, 6807, 7007) has at least a retaining element (6612, 6614, 6826, 6828, 7016), and
- wherein in the receptive condition the shaped element (6608, 6610, 6822, 6824, 7014) is in releasable engagement with the retaining element (6612, 6614, 6826, 6828, 7016).

9. Dispense interface according to any one of claims 4 and 8, wherein the lockout element (6600, 6800, 7000) is configured such that when said dispense interface (6605, 6805, 7005) is attached to said drug delivery device, a distal portion of the drug delivery device acts on said bearing section (6602, 6814, 6816, 7008, 7010) such that said shaped element (6608, 6610, 6822, 6824, 7014) is released out of engagement with said retaining element (6612, 6614, 6826, 6828, 7016).

10. Dispense interface according to any one of claims 1 to 9, wherein the lockout element (6600,6800,7000) is attached to the inner body (6607, 6807, 7007) by a connecting element (6636, 6638, 6808, 6810, 7006).

11. Dispense interface according to any one of claims 2, 3 and 8, wherein the spring element (6624, 6626) and the release section (6604, 6606) adjoin the bearing section (6602) independently.

12. Dispense interface according to any one of claims 2, 3 and 8, wherein the release section (6818, 6820, 7012) adjoins the spring element (6802, 6804, 7002) and wherein the bearing section (6814, 6816, 7008, 7010) is provided on the spring element (6802, 6804, 7002).

13. Dispense interface according to any one of claims 4 and 8, wherein the support section (6830, 6832, 7018) is provided on the release section (6818, 6820, 7012).

14. An apparatus
- comprising a dispense interface (6605,6805,7005) according to any one of the preceding claims, and
- comprising a drug delivery device,
- wherein the dispense interface (6605,6805,7005) is removably attached to the drug delivery device.

## Patentansprüche

1. Abgabeschnittstelle zur Verwendung mit einer Medikamenten-Verabreichungsvorrichtung umfassend
- einen inneren Körper (6607, 6807, 7007) und
- ein Sperrelement (6600, 6800, 7000),
- wobei das Sperrelement (6600, 6800, 7000) an den inneren Körper (6607, 6807, 7007) gekoppelt ist,
- wobei das Sperrelement (6600, 6800, 7000) aus einem Aufnahmezustand in einen gesperrten Zustand beweglich ist,
- wobei die Abgabeschnittstelle (6605, 6805, 7005) im Aufnahmezustand an der MedikamentenVerabreichungsvorrichtung anbringbar ist,
- wobei die Abgabeschnittstelle (6605, 6805, 7005) im Sperrzustand nicht an der Medikamenten-Verabreichungsvorrichtung anbringbar ist,
- wobei das Sperrelement (6600, 6800, 7000) dazu konfiguriert ist, sich aus dem Aufnahmezustand in den gesperrten Zustand zu bewegen, wenn die Abgabeschnittstelle (6605, 6805, 7005) an die Medikamenten-Verabreichungsvorrichtung angebracht und von ihr gelöst wird, **dadurch gekennzeichnet, dass**
- das Sperrelement (6600, 6800, 7000) aus dem Aufnahmezustand in einen aktivierten Zustand beweglich ist und
- wobei das Sperrelement (6600, 6800, 7000) im aktivierten Zustand dazu konfiguriert ist, sich in den gesperrten Zustand zu bewegen, wenn die Abgabeschnittstelle (6605, 6805, 7005) von der Medikamenten-Verabreichungsvorrichtung gelöst wird, und
- wobei das Sperrelement (6600, 6800, 7000) dazu konfiguriert ist, sich aus dem Aufnahmezustand in den aktivierten Zustand zu bewegen, wenn die Abgabeschnittstelle (6605, 6805, 7005) an der Medikamenten-Verabreichungsvorrichtung angebracht wird.

2. Abgabeschnittstelle nach Anspruch 1, wobei das Sperrelement (6600, 6800, 7000) mindestens ein Federelement (6624, 6626, 6802, 6804, 7002) umfasst, das im Aufnahme- und/oder aktivierten Zustand gespannt und im gesperrten Zustand mindestens teilweise entspannt ist.

3. Abgabeschnittstelle nach einem der Ansprüche 1 und 2,
- wobei das Sperrelement (6600, 6800, 7000) mindestens einen Lagerabschnitt (6602, 6814, 6816, 7008, 7010) umfasst, der dazu konfiguriert ist, einen distalen Abschnitt der Medikamenten-Verabreichungsvorrichtung zu tragen,
- wobei der Lagerabschnitt (6602, 6814, 6816, 7008, 7010) im Aufnahmezustand in einer offenen Position ist, in der er dem distalen Abschnitt der Medikamenten-Verabreichungsvorrichtung gestattet, sich dem inneren Körper (6607, 6807, 7007) zu nähern, und
- wobei der Lagerabschnitt (6602, 6814, 6816, 7008, 7010) im gesperrten Zustand in einer Blockierposition ist, in der er den distalen Abschnitt der Medikamenten-Verabreichungsvorrichtung daran hindert, sich dem inneren Körper (6607, 6807, 7007) zu nähern.

4. Abgabeschnittstelle nach Anspruch 3,
- wobei das Sperrelement (6600, 6800, 7000) mindestens einen Stützabschnitt (6616, 6618, 6830, 6832, 7018) umfasst,
- wobei eine Stützfläche (6620, 6622, 6834, 6836, 7020) am inneren Körper (6607, 6807, 7007) oder an einem äußeren Körper (6609, 6809, 7009), der am inneren Körper (6607, 6807, 7007) angebracht ist, vorgesehen ist, und
- wobei das Sperrelement (6600, 6800, 7000) so konfiguriert ist, dass der Stützabschnitt (6616, 6618, 6830, 6832, 7018) im gesperrten Zustand mit der Stützfläche (6620, 6622, 6834, 6836, 7020) in Eingriff steht, um zu verhindern, dass der Lagerabschnitt (6602, 6814, 6816, 7008, 7010) in die offene Position bewegt wird.

5. Abgabeschnittstelle nach einem der Ansprüche 2 und 3, wobei der Lagerabschnitt (6602, 6814, 6816, 7008, 7010) durch das Federelement (6624, 6626, 6802, 6804, 7002) federnd auf dem inneren Körper (6607, 6807, 7007) gestützt ist.

6. Abgabeschnittstelle nach Anspruch 5, wobei das Sperrelement (6600, 6800, 7000) so konfiguriert ist, dass ein distaler Abschnitt der MedikamentenVerabreichungsvorrichtung, wenn die Abgabeschnittstelle (6605, 6805, 7005) an der Medikamenten-Verabreichungsvorrichtung angebracht wird, den Lagerabschnitt (6602, 6814, 6816, 7008, 7010) derart beaufschlagt, dass das Federelement (6624, 6626, 6802, 6804, 7002) gespannt oder weiter gespannt wird.

7. Abgabeschnittstelle nach einem der Ansprüche 5 und 6, wobei das Sperrelement (6600, 6800, 7000) so konfiguriert ist, dass ein distaler Abschnitt der Medikamenten-Verabreichungsvorrichtung, wenn die Abgabeschnittstelle (6605, 6805, 7005) von der Medikamenten-Verabreichungsvorrichtung gelöst wird, vom Lagerabschnitt (6602, 6814, 6816, 7008, 7010) zurückgezogen wird, so dass das Federelement (6624, 6626, 6802, 6804, 7002) mindestens teilweise entspannt und der Lagerabschnitt (6602, 6814, 6816, 7008, 7010) aus der offenen in die Blockierposition bewegt wird.

8. Abgabeschnittstelle nach einem der Ansprüche 1 bis 7,
- wobei das Sperrelement (6600, 6800, 7000) mindestens einen Freigabeabschnitt (6604, 6606, 6818, 6820, 7012) mit einem geformten Element (6608, 6610, 6822, 6824, 7014) hat,
- wobei der innere Körper (6607, 6807, 7007) mindestens ein Halteelement (6612, 6614, 6826, 6828, 7016) hat und
- wobei das geformte Element (6608, 6610, 6822, 6824, 7014) im Aufnahmezustand mit dem Halteelement (6612, 6614, 6826, 6828, 7016) in freigebbarem Eingriff steht.

9. Abgabeschnittstelle nach einem der Ansprüche 4 und 8, wobei das Sperrelement (6600, 6800, 7000) so konfiguriert ist, dass ein distaler Abschnitt der Medikamenten-Verabreichungsvorrichtung, wenn die Abgabeschnittstelle (6605, 6805, 7005) an der Medikamenten-Verabreichungsvorrichtung angebracht wird, den Lagerabschnitt (6602, 6814, 6816, 7008, 7010) so beaufschlagt, dass das geformte Element (6608, 6610, 6822, 6824, 7014) außer Eingriff mit dem Halteelement (6612, 6614, 6826, 6828, 7016) gebracht wird.

10. Abgabeschnittstelle nach einem der Ansprüche 1 bis 9, wobei das Sperrelement (6600, 6800, 7000) über ein Verbindungselement (6636, 6638, 6808, 6810, 7006) am inneren Körper (6607, 6807, 7007) angebracht ist.

11. Abgabeschnittstelle nach einem der Ansprüche 2, 3 und 8, wobei das Federelement (6624, 6626) und der Freigabeabschnitt (6604, 6606) unabhängig an den Lagerabschnitt (6602) anschließen.

12. Abgabeschnittstelle nach einem der Ansprüche 2, 3 und 8, wobei der Freigabeabschnitt (6818, 6820, 7012) an das Federelement (6802, 6804, 7002) anschließt und wobei der Lagerabschnitt (6814, 6816, 7008, 7010) am Federelement (6802, 6804, 7002) vorgesehen ist.

13. Abgabeschnittstelle nach einem der Ansprüche 4 und 8, wobei der Stützabschnitt (6830, 6832, 7018) am Freigabeabschnitt (6818, 6820, 7012) vorgesehen ist.

14. Vorrichtung
- umfassend eine Abgabeschnittstelle (6605, 6805, 7005) nach einem der vorhergehenden Ansprüche und
- umfassend eine Medikamenten-Verabreichungsvorrichtung,
- wobei die Abgabeschnittstelle (6605, 6805, 7005) entfernbar an der Medikamenten-Verabreichungsvorrichtung angebracht ist.

## Revendications

1. Interface de distribution destinée à être utilisée avec un dispositif d'administration de médicament comprenant
- un corps intérieur (6607, 6807, 7007) et
- un élément de verrouillage (6600, 6800, 7000),
- l'élément de verrouillage (6600, 6800, 7000) étant accouplé au corps intérieur (6607, 6807, 7007),
- l'élément de verrouillage (6600, 6800, 7000) étant déplaçable d'un état de réception à un état verrouillé,
- l'interface de distribution (6605, 6805, 7005) pouvant, dans l'état de réception, être attachée au dispositif d'administration de médicament,
- l'interface de distribution (6605, 6805, 7005) ne pouvant pas, dans l'état verrouillé, être attachée au dispositif d'administration de médicament,
- l'élément de verrouillage (6600, 6800, 7000) étant configuré pour se déplacer de l'état de réception à l'état verrouillé lorsque ladite interface de distribution (6605, 6805, 7005) est attachée audit dispositif d'administration de médicament et détachée de celui-ci, **caractérisée en ce que**
- l'élément de verrouillage (6600, 6800, 7000) est déplaçable de l'état de réception à un état activé, et
- l'élément de verrouillage (6600, 6800, 7000) étant, dans l'état activé, configuré pour se déplacer jusqu'à l'état verrouillé lorsque ladite interface de distribution (6605, 6805, 7005) est détachée dudit dispositif d'administration de médicament, et
- l'élément de verrouillage (6600, 6800, 7000) étant configuré pour se déplacer de l'état de réception à l'état activé lorsque ladite interface de distribution (6605, 6805, 7005) est attachée audit dispositif d'administration de médicament.

2. Interface de distribution selon la revendication 1, dans laquelle l'élément de verrouillage (6600, 6800, 7000) comprend au moins un élément formant ressort (6624, 6626, 6802, 6804, 7002), qui est tendu dans l'état de réception et/ou dans l'état activé et est au moins en partie relâché dans l'état verrouillé.

3. Interface de distribution selon l'une quelconque des revendications 1 et 2,
- dans laquelle l'élément de verrouillage (6600, 6800, 7000) comprend au moins une section porteuse (6602, 6814, 6816, 7008, 7010) configurée pour porter une partie distale du dispositif d'administration de médicament,
- dans laquelle, dans l'état de réception, la section porteuse (6602, 6814, 6816, 7008, 7010) se trouve dans une position ouverte, dans laquelle elle permet à la partie distale du dispositif d'administration de médicament de s'approcher du corps intérieur (6607, 6807, 7007), et
- dans laquelle, dans l'état verrouillé, la section porteuse (6602, 6814, 6816, 7008, 7010) se trouve dans une position de blocage, dans laquelle elle empêche la partie distale du dispositif d'administration de médicament de s'approcher du corps intérieur (6607, 6807, 7007).

4. Interface de distribution selon la revendication 3,
- dans laquelle l'élément de verrouillage (6600, 6800, 7000) comprend au moins une section de support (6616, 6618, 6830, 6832, 7018),
- dans laquelle une surface de support (6620, 6622, 6834, 6836, 7020) est prévue sur le corps intérieur (6607, 6807, 7007) ou sur un corps extérieur (6609, 6809, 7009) qui est attaché au corps intérieur (6607, 6807, 7007), et
- dans laquelle l'élément de verrouillage (6600, 6800, 7000) est configuré de telle sorte que, dans l'état verrouillé, la section de support (6616, 6618, 6830, 6832, 7018) soit engrené avec la surface de support (6620, 6622, 6834, 6836, 7020) de façon à empêcher un déplacement de la section porteuse (6602, 6814, 6816, 7008, 7010) jusqu'à la position ouverte.

5. Interface de distribution selon l'une quelconque des revendications 2 et 3, dans laquelle la section porteuse (6602, 6814, 6816, 7008, 7010) est supportée de manière élastique sur le corps intérieur (6607, 6807, 7007) par l'élément formant ressort (6624, 6626, 6802, 6804, 7002).

6. Interface de distribution selon la revendication 5, dans laquelle l'élément de verrouillage (6600, 6800, 7000) est configuré de telle sorte que, lorsque ladite interface de distribution (6605, 6805, 7005) est attachée audit dispositif d'administration de médicament, une partie distale du dispositif d'administration de médicament agisse sur ladite section porteuse (6602, 6814, 6816, 7008, 7010) de telle sorte que ledit élément formant ressort (6624, 6626, 6802, 6804, 7002) soit tendu ou soit tendu en plus.

7. Interface de distribution selon l'une quelconque des revendications 5 et 6, dans laquelle l'élément de verrouillage (6600, 6800, 7000) est configuré de telle sorte que, lorsque ladite interface de distribution (6605, 6805, 7005) est détachée dudit dispositif d'administration de médicament, une partie distale du dispositif d'administration de médicament soit rétractée vis-à-vis de ladite section porteuse (6602, 6814, 6816, 7008, 7010) de telle sorte que ledit élément formant ressort (6624, 6626, 6802, 6804, 7002) soit au moins en partie relâché et ladite section porteuse (6602, 6814, 6816, 7008, 7010) soit déplacée de la position ouverte à la position de blocage.

8. Interface de distribution selon l'une quelconque des revendications 1 à 7,
- dans laquelle l'élément de verrouillage (6600, 6800, 7000) comporte au moins une section de libération (6604, 6606, 6818, 6820, 7012) comprenant un élément profilé (6608, 6610, 6822, 6824, 7014),
- dans laquelle le corps intérieur (6607, 6807, 7007) comporte au moins un élément de retenue (6612, 6614, 6826, 6828, 7016), et
- dans laquelle, dans l'état de réception, l'élément profilé (6608, 6610, 6822, 6824, 7014) est engrené de façon libérable avec l'élément de retenue (6612, 6614, 6826, 6828, 7016).

9. Interface de distribution selon l'une quelconque des revendications 4 et 8, dans laquelle l'élément de verrouillage (6600, 6800, 7000) est configuré de telle sorte que, lorsque ladite interface de distribution (6605, 6805, 7005) est attachée audit dispositif d'administration de médicament, une partie distale du dispositif d'administration de médicament agisse sur ladite section porteuse (6602, 6814, 6816, 7008, 7010) de telle sorte que ledit élément profilé (6608, 6610, 6822, 6824, 7014) soit libéré de l'engrènement dudit élément de retenue (6612, 6614, 6826, 6828, 7016).

10. Interface de distribution selon l'une quelconque des revendications 1 à 9, dans laquelle l'élément de verrouillage (6600, 6800, 7000) est attaché au corps intérieur (6607, 6807, 7007) par le biais d'un élément de raccordement (6636, 6638, 6808, 6810, 7006).

11. Interface de distribution selon l'une quelconque des revendications 2, 3 et 8, dans laquelle l'élément formant ressort (6624, 6626) et la section de libération (6604, 6606) sont reliés à la section porteuse (6602) de façon indépendante.

12. Interface de distribution selon l'une quelconque des revendications 2, 3 et 8, dans laquelle la section de libération (6818, 6820, 7012) est reliée à l'élément formant ressort (6802, 6804, 7002) et dans laquelle la section porteuse (6814, 6816, 7008, 7010) se trouve sur l'élément formant ressort (6802, 6804, 7002).

13. Interface de distribution selon l'une quelconque des revendications 4 et 8, dans laquelle la section de support (6830, 6832, 7018) se trouve sur la section de libération (6818, 6820, 7012).

14. Appareil
- comprenant une interface de distribution (6605, 6805, 7005) selon l'une quelconque des revendications précédentes, et
- comprenant un dispositif d'administration de médicament,
- dans lequel l'interface de distribution (6605, 6805, 7005) est attachée de manière amovible au dispositif d'administration de médicament.
